# EUROPEAN PATENT SPECIFICATION

(11) **EP 3 485 869 B1**
(45) Date of publication and mention of the grant of the patent: **21.07.2021**
(21) Application number: 17202137.0
(22) Date of filing: 16.11.2017
(51) Int. Cl.: A61Q 5/02, A61Q 5/12, A61K 8/34, A61Q 15/00, A61Q 17/00, A61Q 19/00

(54) **USE OF PHYTANTRIOL AS AN ANTIMICROBIAL AGENT IN THE PRESERVATION OF A COMPOSITION**
VERWENDUNG VON PHYTANTRIOL ALS ANTIMIKROBIELLES MITTEL ZUR ERHALTUNG EINER ZUSAMMENSETZUNG
UTILISATION DE PHYTANTRIOL COMME AGENT ANTIMICROBIEN DANS LA CONSERVATION D'UNE COMPOSITION

(43) Date of publication of application: 22.05.2019
(73) Proprietor: DSM IP Assets B.V., 6411 TE Heerlen (NL)
(72) Inventor: MENDROK-EDINGER, Christine, 4303 Kaiseraugst (CH); MONGIAT, Sebastien, 4303 Kaiseraugst (CH); RUDOLPH, Thomas, 4303 Kaiseraugst (CH); SCHLIFKE-POSCHALKO, Alexander, 4303 Kaiseraugst (CH)
(74) Representative: Berg, Katja

(56) References cited:
- EP-A1- 1 529 523
- EP-A2- 1 172 087
- EP-A2- 1 529 519
- WO-A1-2008/110268
- WO-A2-2011/073437
- KR-A- 20090 130 905
- KR-A- 20110 055 829
- US-A1- 2005 131 077

## Description

The present invention relates to the use of phytantriol as antimicrobial agent for improving the preservation of a composition (as laid out in the appended claims).

To protect cosmetic compositions, household products, plastics, paper and/ or paints against mold and bacteria, most products currently on the market contain preservatives. While these preservatives protect against bacteria and fungi, studies have linked daily exposure to many of these substances to an increased risk of skin irritation, cancer and/ or endocrine problems. Thus, many manufactures are searching for alternative antimicrobial actives which allow reducing the amount of preservatives and don't appear to pose any health risks.

Antimicrobial active compounds furthermore play a key role for many cosmetic applications:
Acne is taken to mean a skin disorder which is evident in inflamed papules, pustules or nodules, caused by increased talc production and impaired keratinization of the skin. The inflammation may be associated with reddening, swelling and pressure pain. Besides genetic predisposition, possible causes of acne formation can be androgens, comedogenic substances (for example in cosmetics), smoking, stress or excessive colonization of the skin by bacteria. Acne can be triggered, for example, by microorganisms, such as Propionibacterium acnes, or Staphylococcus epidermidis. Propionibacterium acnes is a bacterium which usually colonizes the skin and lives on sebum. Acne may arise, for example, if the number of these bacteria is increased. The presence of bacteria in the follicles results in inflammation reactions, which is evident in the form of red nodules or pustules. The production of free fatty acids by the bacteria furthermore promotes the inflammation reaction in the follicle.

Besides water and salt, axillary sweat contains many other substances (such as fats, amino acids, sugars, lactic acid, urea, etc.). Freshly formed sweat is odorless; the typical sweat odor only forms due to the action of skin bacteria on the sweat, which decompose the latter. Examples of such bacteria are Staphylococcus or Corynebacterium. For this reason, antimicrobial substances are usually also employed besides aroma substances and antiperspirants in deodorants, with the aim of controlling the bacteria which are involved in the odor formation.

Surprisingly, it has now been found that phytantriol exhibits an antimicrobial activity.

Thus, the present invention relates to the use of phytantriol as antimicrobial agent, i.e. an agent which exhibits an antimicrobial activity. In particular the present invention is directed to the use of phytantriol as anti-fungal and/ or anti-bacterial agent, more in particular as an agent for killing and/ or inhibiting the growth of fungi and/ or gram-positive bacteria such as in particular Propionibacterium acnes (P. acnes), Staphylococcus epidermis (S. epidermis), Malessazia furfur (M. furfur), Aspergillus brasiliensis (A. brasiliensis), Candida albicans (C. albicans) and/ or Staphylococcus aureus (S. aureus).

In another embodiment, the invention relates to a method for killing and/ or inhibiting growth of microbial cells, in particular fungal and/ or bacterial cells, said method comprising contacting said microbial cells with phytantriol. In a preferred embodiment, the microbial cells are selected from the group consisting of fungi and/or gram-positive bacteria, more preferably from the group consisting of Propionibacterium acnes (P. acnes), Staphylococcus epidermis (S. epidermis), Malessazia furfur (M. furfur), Aspergillus brasiliensis (A. brasiliensis), Candida albicans (C. albicans), and Staphylococcus aureus (S. aureus) as well as mixtures thereof.

Phytantriol [CAS: 74563-64-7] is a colourless to light yellow, viscous liquid with the chemical name 3,7,11,15-tetramethyl-hexadecane-1,2,3-triol. Phytantriol is e.g. commercially available at DSM Nutritional Products Ltd, Kaiseraugst.

The term "antimicrobial activity" (or "antimicrobial effect") as used herein means a capability of killing and/or inhibiting the growth of microbial cells such as in particular bacteria and fungi and more in particular P. acnes, S. epidermis, M. furfur, A. brasiliensis, C. albicans and S. aureus as well as mixtures thereof.

Due to the antimicrobial activity, phytantriol is also suitable to maintain skin homeostasis and/ or balance the skin microbiome by treating overpopulation of microorganisms on the skin such as P. acnes (acne control application) and S. epidermis (antiperspirant/ deodorant applications) and/or reducing unwanted microorganisms such as S. aureus.

In all embodiments of the present invention phytantriol as an antimicrobial agent is preferably used in an amount selected in the range of about 0.005 to 2 wt.-%, preferably 0.01 to 1 wt.-%, more preferably in the range of about 0.05 to 0.75 wt.-% and most preferably in the range of 0.1 to 0.5 wt.-%, based on the total weight of the composition.

To make use of the anti-microbial activity of phytantriol, it can be used in a multiplicity of formulations or applications, such as, for example, cosmetic or pharmaceutical compositions, medicinal products, household products, plastics, plastisols, paper and/or paints.

The invention relates to a method of preventing microbial decay and breakdown of cosmetic and/ or pharmaceutical compositions, household products, plastics, paper and/ or paints, wherein said method comprises adding to the compositions, products, plastics, papers and/ or paints phytantriol in an amount of 0.005 to 2 wt.-%, more preferably in the range of about 0.01 to 1 wt.-%, most preferably in the range of about 0.05 to 0.75 wt.-% such as in the range of 0.1 to 0.5 wt.-% as an antimicrobial agent. In a particular embodiment, the method also encompasses the step of appreciating the result.

In a further embodiment, the invention also relates to a method of preserving a cosmetic or pharmaceutical composition against microbiological contamination or growth, wherein said method comprises adding to the compositions, products, plastics, papers and/ or paints phytantriol in an amount of 0.005 to 2 wt.-%, more preferably in the range of about 0.01 to 1 wt.-%, most preferably in the range of about 0.05 to 0.75 wt.-% such as in the range of 0.1 to 0.5 wt.-% as an antimicrobial agent.

In a particular advantageous embodiment, the invention relates to a method of preventing microbial decay and breakdown of cosmetic or pharmaceutical compositions furthermore comprising water and at least one further agent selected from the group consisting of surfactants, emulsifiers, thickeners, and oils as such compositions are particular sensitive to microbial growth.

Thus, in another embodiment, the invention is also directed to cosmetic or pharmaceutical compositions comprising water and at least one agent selected from the group consisting of surfactants, emulsifiers, thickeners and oils, wherein the composition furthermore comprises phytantriol in an amount of 0.005 to 2 wt.-%, more preferably in the range of about 0.01 to 1 wt.-%, most preferably in the range of about 0.05 to 0.75 wt.-% such as in the range of 0.1 to 0.5 wt.-%, based on the total weight of the composition.

Also disclosed is the use of phytantriol as anti-acne, deodorant or antiperspirant active compound. In particular, phytantriol is suitable for the treatment or prophylaxis of acne which is triggered by P. Acnes or S. epidermidis.

Also disclosed is the use of phytantriol as active compound in deodorants or antiperspirants as it has an antimicrobial action against the bacteria which are responsible for the decomposition of sweat and thus for the formation of the odour, i.e. S. epidermis.

Advantageously, phytantriol can also be used in combination with traditional preservatives to improve the preservative activity thereof.

The use according to the invention of phytantriol can take place both in the cosmetic sense and in the pharmaceutical sense. A pharmaceutical application is conceivable, for example, in the case of anti-acne compositions. In all embodiments of the present invention, the use is however preferably cosmetic (non-therapeutic).

The cosmetic or pharmaceutical compositions according to the present invention are in particular topically applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

Suitable surfactants, emulsifiers, thickeners, and oils for the purpose of the present inventions are alls surfactants, emulsifiers, thickeners, and oils commonly used in cosmetic applications and which are e.g. listed in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The compositions according to the present invention are generally prepared by admixing phytantriol in an amount selected in the range 0.005 to 2 wt.-%, more preferably in the range of about 0.01 to 1 wt.-%, most preferably in the range of about 0.05 to 0.75 wt.-% such as in the range of 0.1 to 0.5 wt.-%, based on the total weight of the composition with a suitable carrier.

The cosmetic or pharmaceutical compositions according to the present invention preferably further comprise a physiologically acceptable medium, that is to say a medium compatible with keratinous substances, such as the skin, mucosa, and keratinous fibres. In particular the physiologically acceptable medium is a cosmetically or pharmaceutically acceptable carrier.

The term cosmetically or pharmaceutically acceptable carrier refers to all carriers and/or excipients and/ or diluents conventionally used in cosmetic compositions or pharmaceutical compositions.

Preferably, the cosmetic or pharmaceutical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

The cosmetic or pharmaceutical compositions in accordance with the invention can be in the form of a liquid, lotion, a thickened lotion, a gel, a cream, a milk, an ointment or a paste.

The cosmetic or pharmaceutical compositions according to the invention have a pH in the range of 3-10, preferably in the range of pH of 3-8, most preferred in the range of pH 3.5-7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention the acid, if present, is used in an amount of at least 0.0001 wt.-%, such as e.g. in an amount of 0.01-1 wt.-%, in particular in an amount of 0.01 to 0.5 wt.-%.

The cosmetic compositions according to the present invention are in particular skin care preparations, functional preparations and/ or hair care preparations such as most in particularly skin or hair care preparations.

Examples of skin care preparations are, in particular, light protective preparations (sun care preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Preferably in all embodiments of the present invention the skin care preparation is a deodorant, an anti-perspirant, or an anti-acne composition.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples of hair care preparations which are suitable according to the invention and which may be mentioned are shampoos, hair conditioners (also referred to as hair rinses), hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

If the hair care preparations according to the invention are supplied as shampoos, these can be clear liquids, opaque liquids (with pearly luster effect), in cream form, gel-like or else in powder form or in tablet form, and as aerosols. The surfactant raw materials on which these shampoos are based can be anionic, cationic, nonionic and amphoteric in nature and also be present in combinations of these substances.

Examples of anionic surfactants suitable for the incorporation into the shampoo preparations according to the present invention are C₁₀₋₂₀ alkyl- and alkylenecarboxylates, alkyl ether carboxylates, fatty alcohol sulfates, fatty alcohol ether sulfates, alkylolamide sulfates and sulfonates, fatty acid alkylolamide polyglycol ether sulfates, alkanesulfonates and hydroxyalkanesulfonates, olefinsulfonates, acyl esters of isothionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyltaurides, fatty acid sarcosinates, and sulforicinoleates. These compounds and their mixtures are used in the form of their salts which are soluble in water or dispersible in water, for example the sodium, potassium, magnesium, ammonium, mono-, di- and triethanolammonium and analogous alkylanunonium salts.

Examples of suitable cationic surfactants are quaternary ammonium salts such as di(C₁₀₋-C₂₄alkyl)dimethylammonium chloride or bromide, preferably di (C₁₂-C₁₈alkyl)-dimethylammonium chloride or bromide; C₁₀-C₂₄-alkyldimethylethylammonium chloride or bromide; C₁₀-C₂₄-alkyltrimethylammonium chloride or bromide, preferably cetyltrimethylammonium chloride or bromide and C₂₀-C₂₄-alkyltrimethylammonium chloride or bromide; C₁₀-C₂₄4 -alkyldimethylbenzylammonium chloride or bromide, preferably C₁₂-C₁₈-alkyldime methylbenzylammoniumchloride; N-(C₁₂-C₁₈-alkyl)pyridinium chloride or bromide, preferably N- (C₁₂-C₁₆-alkyl)pyridinium chloride or bromide; N-(C₁₂-C₁₈-alkyl)isoquinolinium chloride, bromide or monoalkyl sulfate; N-(C₁₂-C₁₈-alkyloylcolaminoformylmethyl)pyridinium chloride; N-(C₁₂-C₁₈-alkyl)-N-methylmorpholinium chloride, bromide or monoalkyl sulfate; N-(C₁₂-C₁₈-alkyl)-N-ethylmorpholinium chloride, bromide or monoalkyl sulfate; C₁₆-C₁₈-alkylpentaoxethylammonium chloride; isobutylphenoxyethoxyethyldimethyl-benzylammonium chloride; salts of N,N-diethylaminoethylstearylamide and oleylamide with hydrochloric acid, acetic acid, lactic acid, citric acid, phosphoric acid; N-acylamidoethyl-N,N-diethyl-N-methylammonium chloride, bromide or monoalkylsulfate and N-acylaminoethyl-N,N-diethyl-N-benzylammonium chloride, bromide or monoalkyl sulfate, where acyl is preferably stearyl or oleyl.

Examples of suitable nonionic surfactants which can be used as detergent substances are fatty alcohol ethoxylates (alkylpolyethylene glycols); alkylphenol polyethylene glycols; alkyl mercaptan polyethylene glycols; fattyamine ethoxylates (alkylaminopolyethylene glycols); fatty acid ethoxylates (acylpolyethylene glycols); polypropylene glycol ethoxylates (Pluronic); fatty acid alkylolamides (fatty acid amide polyethylene glycols); sucrose esters; sorbitol esters and polyglycol ether.

Examples of amphoteric surfactants which can be added to the shampoos are N-(C₁₂-C₁₈-alkyl)-.beta.-aminopropionates and N-(C₁₂-C₁₈-alkyl)-.beta.-iminodipropionates as alkali metal and mono-, di- and trialkylammonium salts; N-acylamidoalkyl-N,N-dimethylacetobetaine, preferably N-(C₈-C₁₈-acyl)amidopropyl-N, N-dimethylacetobetaine; C₁₂-C₁₈-alkyldimethylsulfopropylbetaine; amphoteric surfactants based on imidazoline (commercial name: Miranol®, Steinapon®), preferably the sodium salt of 1-(β-carboxymethyloxyethyl)-1-(carboxymethyl)-2-laurylimidazolinium; amine oxide, for example C₁₂-C₁₈-alkyldimethylamine oxide, fatty acid amidoalkyldimethylamine oxide.

The hair care preparations according to the invention can additionally contain further additives customary in hair care such as for example perfumes, colorants, also those which simultaneously dye or tint the hair, solvents, opacifying agents and pearly luster agents, for example esters of fatty acids with polyols, magnesium and zinc salts of fatty acids, dispersions based on copolymers, thickening agents such as sodium, potassium and ammonium chloride, sodium sulfate, fatty acid alkylolamides, cellulose derivatives, natural rubbers, also plant extracts, protein derivatives such as gelatin, collagen hydrolysates, polypeptides with a natural or synthetic basis, egg yolk, lecithin, lanolin and lanolin derivatives, fats, oils, fatty alcohols, silicones, deodorizing agents, substances with antimicrobial activity, substances with antiseborrhoeic activity, substances with keratolytic and keratoplastic effect, such as, for example, sulfur, salicylic acid and enzymes as well as further anti-dandruff agents such as olamine, climbazol, zink pyrithion, ketoconazole, salicylic acid, sulfur, tar preparations, derivatives of undecenic acid, extracts of nettel, rosmary, cottonwood, birch, walnut, willow bark and/ or arnica.

For the preparation of the hair care preparations the phytantriol is dissolved under stirring at a temperature in the range between 20 and 40°C, preferably at room temperature. Subsequently, the further additives are added.

In the event of alcohol containing scalp respectively hair care preparations phytantriol is dissolved in the alcohol at a temperature in the range between 20 and 40°C, preferably at room temperature. Subsequently, the further additives are added.
In the event of hair rinses and oil-in-water emulsions the active substance is added to the final emulsion below 40°C under stirring.

The shampoos are produced in a manner known per se by mixing the individual components and where necessary further processing appropriate for the particular type of preparation.

Examples of hair care preparations in which the phytantriol can be used according to the invention and which may be mentioned are hair conditioners, hair tonics and hair regenerating compositions, which are rinsed off from the hair after a certain time or, depending on the formulation, can also remain on the hair. These products contain, inter alia, substances from the group of the above mentioned cationic substances which display a reviving and antistatic property on the hair.

All these preparations are also produced as already mentioned for the shampoo in a manner known per se with the addition of the phytantriol.

Particular suitable hair care preparations according to the present invention are shampoo preparations comprising (i) phytantriol in an amount selected in the range of 0.005 to 0.5 wt.-%, more preferably in the range of about 0.01 to 0.2 wt.-%, most preferably in the range of about 0.05 to 0.1 wt.-%, based on the total weight of the composition, (ii) water and (iii) at least one anionic surfactant. Preferably, the anionic surfactant is selected from the group consisting of sodium lauryl sulfate, ammonium lauryl sulfate, sodium lauryl ether sulfate, ammonium lauryl ether sulfate, sodium lauroyl sarconisate, sodium oleylsuccinate, ammonium lauryl sulfosuccinate, sodium dodecylbenzol sulfonate and/ or triethanolamine dodecylbenzol sulfonate or mixtures thereof, such as in particular sodium lauryl sulfate, ammonium lauryl sulfate, sodium lauryl ether sulfate and/ or ammonium lauryl ether sulfate. The total amount of the anionic surfactant in the compositions according to the invention ranges from 0.5 to 45 wt.-%, preferably from 1.5 to 35 wt.-%, more preferably from 7 to 25 wt.-%, in particular from 7 to 15 wt.-% based on the total weight of the composition.

Particular suitable hair conditioners according to the present invention may be rinse off or leave on conditioners, preferably rinse-off conditioners. Particular advantageous hair conditioners according to the present invention comprise (i) phytantriol in an amount selected in the range of 0.005 to 1 wt.-%, more preferably in the range of about 0.01 to 5 wt.-%, most preferably in the range of about 0.05 to 0.2 wt.-%, based on the total weight of the composition, (ii) water and (iii) at least one conditioning agent such as e.g. silicone oils, quaternary polymers, naturally derived conditioning agents, etc.

The quaternary polymer is preferably selected from e.g. Polyquaternium-6 (e.g. commercialized under the trade name TILAMAR® Quat 640 or 641), Polyquaternium-22 (e.g. commercialized under the trade name TILAMAR® Quat 2240 or 2241), Polyquaternium-7 (e.g. commercialized under the trade name TILAMAR® Quat 710, 711 or 712), etc, The naturally derived conditioning agents are preferably selected from e.g. sugar based polymers such as Guar Hydroxypropyltrimonium Chloride (e.g. commercialized under the trade name Jaguar C-17, Jaguar C-1000, Jaguar C-13S), but not limited hereto.

In principle any silicone oil is suitable for use in the hair conditioner. However, the silicone oil is preferably selected from dimethicones, dimethiconols, polydimethylsiloxanes, arylated silicones, cyclic silicones, silicone surfactants and aminated silicones and may be volatile or non-volatile. Particular suitable silicone oils are dimethicone, dimethiconol, polydimethylsiloxane which are available from various suppliers such as Dow Corning. The total amount of the at least one silicone oil and/or quaternary polymer and/or naturally derived conditioning agent in the hair conditioner is preferably selected is in the range of 0.01 to 10 wt.-%, preferably 0.02 to 7.5 wt.-%, more preferably 0.05 to 5 wt.-% and most preferably 0.1 to 3 wt.-%, based on the total weight of the composition.

In another preferred embodiment, the cosmetic compositions according to the present invention are O/W emulsions, W/O emulsions and/ or gels such as shower gels or hair gels.

The O/W emulsions according to the present invention advantageously comprise (i) phytantriol in an amount selected in the range of 0.005 to 2 wt. %, more preferably in the range of about 0.01 to 1 wt. %, most preferably in the range of about 0.05 to 0.75 wt.-% such as in the range of 0.1 to 0.5 wt.-%, based on the total weight of the composition, (ii) water and (iii) at least one O/W- or Si/W-emulsifier selected from the list of glycerylstearatcitrate, glycerylstearate (self-emulsifying), stearic acid, salts of stearic acid, polyglyceryl-3-methylglycosedistearate, ceteareth-20, steareth-2, steareth-12, PEG-40 stearate, phosphate esters and the salts thereof such as cetyl phosphate (Amphisol® A), diethanolamine cetyl phosphate (Amphisol® DEA), potassium cetyl phosphate (Amphisol® K), sodiumcetearylsulfat, sodium glyceryl oleate phosphate, hydrogenated vegetable glycerides phosphate, sorbitan oleate, sorbitan sesquioleate, sorbitan isostearate, sorbitan trioleate, lauryl glucoside, decyl glucoside, sodium stearoyl glutamate, sucrose polystearate and Hydrated Polyisobuten as well as mixtures thereof. Also, one or more synthetic polymers may be used as an emulsifier such as for example, PVP eicosene copolymer, acrylates/C10-3o alkyl acrylate crosspolymer, acrylates/steareth-20 methacrylate copolymer, PEG-22/dodecyl glycol copolymer, PEG-45/dodecyl glycol copolymer, and mixtures thereof. In a particular preferred embodiment the O/W-emulsifier is selected from the group of cetyl phosphates such as in particular potassium cetyl phosphate (commercially available as Amphisol® K), glyceryl stearate (and) PEG-100 stearate (commercially available as Arlacel® 165), Polyacrylamide, Sodium Polyacrylate, Sodium Polyacryloyldimethyl Taurate, Decyl Glucoside, Ceteral Glucoside, Caprylyl/Capryl Glucoside, Sorbitan Olivate, Polygelyceryl-4 Olivate, Polyglyceryl-3 Methylglucose Distearate, Polysorbate 20, Polysorbate 60 and/ or polyalkylenglycolether such as in particular laureth-35 (lauryl alcohol with 35 EO units; commercially available as Brij® 35). The at least one O/W emulsifier is preferably used in an amount of about 0.001 to 10 wt.-%, more preferably in an amount of 0.1 to 7 wt.-% with respect to the total weigh of the composition. Additionally, the cosmetic composition in the form of a O/W emulsion contains advantageously at least one co-emulsifier selected from the list of alkyl alcohols such as Cetyl Alcohol (Lorol C16, Lanette 16) Cetearyl Alcohol (Lanette® O), Stearyl Alcohol (Lanette® 18), Behenyl Alcohol (Lanette® 22), Glyceryl Monostearate, Glyceryl Myristate (Estol® 3650), Hydrogenated Coco- Glycerides (Lipocire Na10) without being limited to this and mixtures thereof.

The W/O emulsions according to the present invention advantageously comprise (i) phytantriol in an amount selected in the range of 0.005 to 2 wt.-%, more preferably in the range of about 0.01 to 1 wt.-%, most preferably in the range of about 0.05 to 0.75 wt.-% such as in the range of 0.1 to 0.5 wt.-%, based on the total weight of the composition, (ii) water and (iii) at least one W/O- or W/Si-emulsifier selected from the list of polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The gel preparations according to the present invention advantageously comprise (i) phytantriol in an amount selected in the range of 0.005 to 2 wt.-%, more preferably in the range of about 0.01 to 1 wt.-%, most preferably in the range of about 0.05 to 0.75 wt.-% such as in the range of 0.1 to 0.5 wt.-%, based on the total weight of the composition, (ii) water and (iii) at least one water soluble thickener. Such water-soluble thickeners are well known to a person skilled in the art and are e.g. listed in the "Handbook of Water soluble gums and resins" by Robert L. Davidson (Mc Graw Hill Book Company (1980)). Particularly suitable water soluble thickeners are selected from the group consisting of polyacrylic acids (e.g. commercially available under the tradename Carbomer or Carbopol®), homopolymers of 2-Acrylamido-2-methylpropansulfonic acid (e.g. commercially available as Rheothik®11-80), acrylate copolymers (e.g. commercially available under the tradename Pemulen® or Aculyn® 33), branched Poly(methacryloyloxyethyltrimethylammoniumchlorid) (INCI-name Polyquaternium-37), non-modified guar gums (e.g. commercially available under the tradename Jaguar), starch or derivatives thereof and/ or hydroxyalkylcellulosen. Preferably the water-soluble thickener is used in an amount of about 0.001 to 10 wt.-%, more preferably in an amount of 0.1 wt.-% to 7 wt.-%, based on the total weigh of the composition.

The following examples are provided to further illustrate the compositions and effects of the present invention. These examples are illustrative only and are not intended to limit the scope of the invention in any way.

### Example 1: Antimicrobial Efficacy

The antimicrobial efficacy is assessed in analogy to the regulatory challenge test method (NF EN ISO11930). Thus, a solution of 0.2 wt.-% of phytantriol in a physiological serum with 0.85 wt.-% NaCl supplemented with 10 wt.-% ethanol was prepared. A control was also prepared under sterile conditions based on a serum with 0.85 wt.-% NaCl and 10 wt.-% ethanol.

The control as well as the phytantriol solution were deposed in 96-deep well plates (1.6 ml/well). The wells were contaminated with the respective bacterial or the fungal strains as outlined in table 1 at 2.5*10⁵ to 5.6*10⁵ cfu/ml for the bacteria and 1*10⁴ to 2.5*10⁴ cfu/ml for the fungi to obtain the initial contamination as outlined in table 1. After the contamination, each well was thoroughly mixed to ensure a homogeneous distribution of the microorganism. Then each plate was incubated at 22°C for 24h. The counting of the (remaining) population was carried out 24h after contamination.

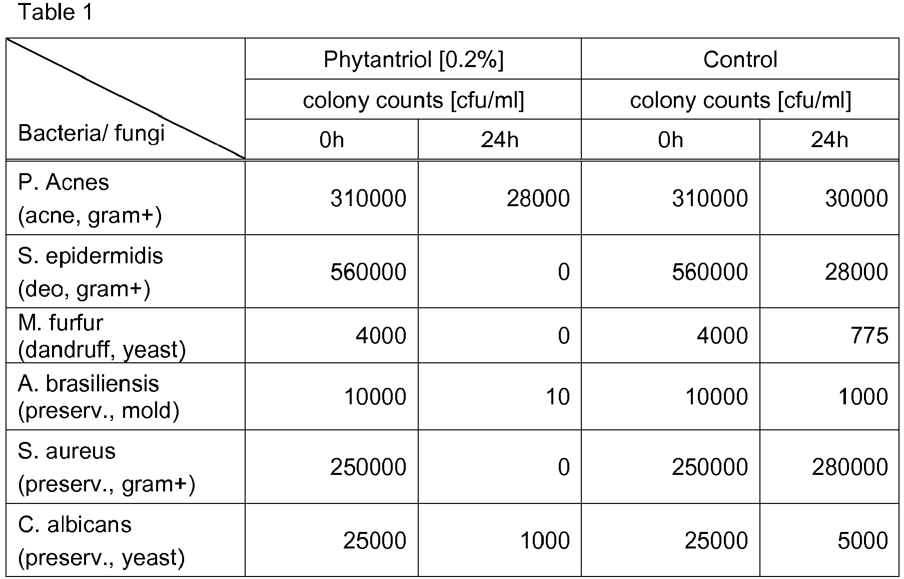

As can be seen in the table above phytantriol has a significant antimicrobial effect against Candida albicans, Staphylococcus aureus, Aspergillus brasiliensis, Staphylococcus epidermidis, Malassezia furfur and Propionibacterium acnes.

## Claims

1. Use of phytantriol as an antimicrobial agent for improving preservation.

2. The use according to claim 1, wherein the antimicrobial agent is an antifungal and/ or antibacterial agent.

3. The use according to claim 2, wherein the antifungal and/ or antibacterial agent is an agent that inhibits the growth of P. acnes, S. epidermis, M. furfur, A. brasiliensis C. albicans and S. aureus as well as mixtures thereof.

4. The use according to any one of claims 1 to 3 in cosmetic compositions, household products, plastics, paper and/ or paints.

5. A method of preventing microbial decay and breakdown of cosmetic and/ or pharmaceutical compositions, household products, plastics, paper and or paints, wherein said method comprises adding to the cosmetic and/ or pharmaceutical compositions, household products, plastics, paper and/ or paints phytantriol as an antimicrobial agent in an amount selected in the range of 0.005 to 2 wt.-%, based on the total weight of the respective composition, household product, plastic, paper and/ or paint.

6. The method according to claim 5, wherein the amount of phytantriol is selected in the range of 0.01 to 1 wt.-%, based on the total weight of the respective composition, household product, plastic, paper and/ or paint.

7. The method according to claim 5, wherein the amount of phytantriol is selected in the range of 0.05 to 0.75 wt.-%, based on the total weight of the composition.

8. The method according to claim 5, wherein the amount of phytantriol is selected in the range of 0.1 to 0.5 wt.-%, based on the total weight of the composition.

9. The method according to any one of claims 5 to 8, wherein the composition furthermore
comprises water and at least one agent selected from the group consisting of surfactants, emulsifiers, thickeners and oils.

10. The method according to any one of claims 5 to 9, wherein the composition is a
cosmetic composition in the form of a shampoo preparation, a hair conditioner, an O/W emulsion, a W/O emulsion or a gel.

## Patentansprüche

1. Verwendung von Phytantriol als antimikrobielles Mittel zur Verbesserung der Konservierung.

2. Verwendung nach Anspruch 1, wobei es sich bei dem antimikrobiellen Mittel um ein antimykotisches und/oder antibakterielles Mittel handelt.

3. Verwendung nach Anspruch 2, wobei es sich bei dem antimykotischen und/oder antibakteriellen Mittel um ein Mittel handelt, das das Wachstum von P. acnes, S. epidermis, M. furfur, A. brasiliensis, C. albicans und S. aureus sowie Gemischen davon inhibiert.

4. Verwendung nach einem der Ansprüche 1 bis 3 in kosmetischen Zusammensetzungen, Haushaltsprodukten, Kunststoffen, Papier und/oder Anstrichmitteln.

5. Verfahren zur Prävention von mikrobiellem Zerfall und Abbau von kosmetischen und/oder pharmazeutischen Zusammensetzungen, Haushaltsprodukten, Kunststoffen, Papier und/oder Anstrichmitteln, bei dem man den kosmetischen und/oder pharmazeutischen Zusammensetzungen, den Haushaltsprodukten, den Kunststoffen, dem Papier und/oder den Anstrichmitteln Phytantriol als antimikrobielles Mittel in einer Menge, die im Bereich von 0,005 bis 2 Gew.-%, bezogen auf das Gesamtgewicht der jeweiligen Zusammensetzung, des jeweiligen Haushaltsprodukts, des jeweiligen Kunststoffs, des jeweiligen Papiers und/oder des jeweiligen Anstrichmittels, gewählt wird, zusetzt.

6. Verfahren nach Anspruch 5, wobei die Menge von Phytantriol im Bereich von 0,01 bis 1 Gew.-%, bezogen auf das Gesamtgewicht der jeweiligen Zusammensetzung, des jeweiligen Haushaltsprodukts, des jeweiligen Kunststoffs, des jeweiligen Papiers und/oder des jeweiligen Anstrichmittels, gewählt wird.

7. Verfahren nach Anspruch 5, wobei die Menge von Phytantriol im Bereich von 0,05 bis 0,75 Gew.-%, bezogen auf das Gesamtgewicht der Zusammensetzung, gewählt wird.

8. Verfahren nach Anspruch 5, wobei die Menge von Phytantriol im Bereich von 0,1 bis 0,5 Gew.-%, bezogen auf das Gesamtgewicht der Zusammensetzung, gewählt wird.

9. Verfahren nach einem der Ansprüche 5 bis 8, wobei die Zusammensetzung ferner Wasser und mindestens ein Mittel aus der Gruppe bestehend aus Tensiden, Emulgatoren, Verdickern und Ölen umfasst.

10. Verfahren nach einem der Ansprüche 5 bis 9, wobei es sich bei der Zusammensetzung um eine kosmetische Zusammensetzung in Form einer Shampoozubereitung, eines Haarkonditionierungsmittels, einer O/W-Emulsion, einer W/O-Emulsion oder eines Gels handelt.

## Revendications

1. Utilisation de phytantriol comme agent antimicrobien pour l'amélioration de la conservation.

2. Utilisation selon la revendication 1, l'agent antimicrobien étant un agent antifongique et/ou antibactérien.

3. Utilisation selon la revendication 2, l'agent antifongique et/ou antibactérien étant un agent qui inhibe la croissance de P. acnes, S. epidermis, M. furfur, A. brasiliensis, C. albicans et S.aureus ainsi que des mélanges correspondants.

4. Utilisation selon l'une quelconque des revendications 1 à 3 dans des compositions cosmétiques, des produits ménagers, des plastiques, du papier et/ou des peintures.

5. Procédé de prévention de la dégradation et de la décomposition microbienne de compositions cosmétiques et/ou pharmaceutiques, de produits ménagers, de plastiques, de papier et/ou de peintures, ledit procédé comprenant l'ajout aux compositions cosmétiques et/ou pharmaceutiques, aux produits ménagers, aux plastiques, au papier et/ou aux peintures, de phytantriol en tant qu'agent antimicrobien en une quantité choisie dans la plage de 0,005 à 2 % en poids, sur la base du poids total de la composition respective, du produit ménager, du plastique, du papier et/ou de la peinture.

6. Procédé selon la revendication 5, la quantité de phytantriol étant choisie dans la plage de 0,01 à 1 % en poids, sur la base du poids total de la composition respective, du produit ménager, du plastique, du papier et/ou de la peinture.

7. Procédé selon la revendication 5, la quantité de phytantriol étant choisie dans la plage de 0,05 à 0,75 % en poids, sur la base du poids total de la composition.

8. Procédé selon la revendication 5, la quantité de phytantriol étant choisie dans la plage de 0,1 à 0,5 % en poids, sur la base du poids total de la composition.

9. Procédé selon l'une quelconque des revendications 5 à 8, la composition comprenant en outre de l'eau et au moins un agent choisi dans le groupe constitué par des tensioactifs, des émulsifiants, des épaississants et des huiles.

10. Procédé selon l'une quelconque des revendications 5 à 9, la composition étant une composition cosmétique sous forme d'une préparation de shampooing, d'un après-shampooing, d'une émulsion huile/eau, d'une émulsion eau/huile ou d'un gel.
